# EUROPEAN PATENT APPLICATION

(11) **EP 1 138 768 A2**
(43) Date of publication of application: **04.10.2001**
(21) Application number: 01201137.5
(22) Date of filing: 27.03.2001
(51) Int. Cl.: C12N 15/12, C07K 14/47, C07K 16/18, A61K 38/17, A61K 48/00

(54) **Apoptin-associating protein AAP-5**

(30) Priority: 27.03.2000 EP 00201108
(71) Applicant: Leadd B.V., 2333 AL Leiden (NL)
(72) Inventor: Noteborn, Mathieu Hubertus Maria, 2352 EH Leiderdorp (NL); Rohn, Jennifer Leigh, 1074 BR Amsterdam (NL); Danen-van Oorschot, Astrid Adriana Anna Maria, 2651 VG Berkel en Rodenrijs (NL)
(74) Representative: Prins, Adrianus Willem

(57) **Abstract**

The invention relates to the field of apoptosis. The invention provides novel therapeutic possibilities, for example novel combinatorial therapies or novel therapeutic compounds that can work alone, sequentially to, or jointly with Apoptin, especially in those cases wherein p53 is (partly) non-functional.

### Annex to the application documents - subsequently filed sequences listing

SEQUENCE LISTING

<110> Leadd B.V.

<120> Apoptin-associating protein

<130> P52887EP10

<140> 01201137.5
<141> 2001-03-27

<150> EP 00201108.8
<151> 2000-03-27

<160> 27

<170> PatentIn Ver. 2.1

<210> 1
<211> 17
<212> DNA
<213> Artificial Sequence

<220>
<223> Description of Artificial Sequence: pACT-specific
      primer

<220>
<221> misc_feature
<222> (1)..(17)

<400> 1
taccactaca atggatg
17

<210> 2
<211> 16
<212> PRT
<213> Artificial Sequence

<220>
<223> Description of Artificial Sequence: peptides for
      AAP-5 antibody production

<220>
<221> SITE
<222> (1)..(16)

<400> 2
Cys Gly Gly Ala Thr His Val Tyr Arg Tyr His Arg Gly Glu
Ser Lys
  1               5                   10
15

<210> 3
<211> 16

<212> PRT
<213> Artificial Sequence

<220>
<223> Description of Artificial Sequence: peptides for
      AAP-5 antibody production

<220>
<221> SITE
<222> (1)..(16)

<400> 3
Gly Asn Gly Gln Arg Lys Asp Arg Lys Lys Thr Ser Leu Gly
Pro Cys
  1               5                   10
15


<210> 4
<211> 16
<212> PRT
<213> Artificial Sequence

<220>
<223> Description of Artificial Sequence: peptides for
      AAP-5 antibody production

<220>
<221> SITE
<222> (1)..(16)

<400> 4
Glu His Ala Pro Glu Ala Ser Gln Pro Ala Glu Asn Ile Ser
Lys Cys
  1               5                   10
15


<210> 5
<211> 28
<212> DNA
<213> Artificial Sequence

<220>
<223> Description of Artificial Sequence: RT-PCR AAP-5
      5' primer

<220>
<221> misc_feature
<222> (1)..(28)

<400> 5
ggagccatgg acaactgttt ggcggccg
28


<210> 6
<211> 28

<212> DNA
<213> Artificial Sequence

<220>
<223> Description of Artificial Sequence: RT-PCR AAP5 3'
      primer

<220>
<221> misc_feature
<222> (1)..(28)

<400> 6
gtgatggcag tgatggtcaa catcacac
28


<210> 7
<211> 23
<212> DNA
<213> Artificial Sequence

<220>
<223> Description of Artificial Sequence: PCR #5F sense
      primer

<220>
<221> misc_feature
<222> (1)..(23)

<400> 7
atattattca tctgtgccag agg
23


<210> 8
<211> 23
<212> DNA
<213> Artificial Sequence

<220>
<223> Description of Artificial Sequence: PCR #5R
      antisense primer

<220>
<221> misc_feature
<222> (1)..(23)

<400> 8
cctctggcac agatgaataa tat
23


<210> 9
<211> 15
<212> PRT
<213> Artificial Sequence

<220>

<223> Description of Artificial Sequence: peptides to
      generate polyclonal antibodies

<220>
<221> SITE
<222> (1)..(15)

<400> 9
Ile Tyr Gln Arg Ser Gly Glu Arg Pro Val Thr Ala Gly Glu
Glu
  1               5                   10
15


<210> 10
<211> 15
<212> PRT
<213> Artificial Sequence

<220>
<223> Description of Artificial Sequence: peptides to
      generate polyclonal antibodies

<220>
<221> SITE
<222> (1)..(15)

<400> 10
Asp Glu Gln Val Pro Asp Ser Ile Asp Ala Arg Glu Ile Phe
Asp
  1               5                   10
15


<210> 11
<211> 15
<212> PRT
<213> Artificial Sequence

<220>
<223> Description of Artificial Sequence: peptides to
      generate polyclonal antibodies

<220>
<221> SITE
<222> (1)..(15)

<400> 11
Arg Ser Ile Asn Asp Pro Glu His Pro Leu Thr Leu Glu Glu
Leu
  1               5                   10
15


<210> 12
<211> 15
<212> PRT
<213> Artificial Sequence

<220>
<223> Description of Artificial Sequence: peptides to
      generate polyclonal antibodies

<220>
<221> SITE
<222> (1)..(15)

<400> 12
Glu Glu Ser Thr Pro Val His Asp Ser Pro Gly Lys Asp Asp
Ala
  1               5                   10
15


<210> 13
<211> 15
<212> PRT
<213> Artificial Sequence

<220>
<223> Description of Artificial Sequence: peptides to
      generate polyclonal antibodies

<220>
<221> SITE
<222> (1)..(15)

<400> 13
Asp Ser Phe Lys Thr Lys Asp Ser Phe Arg Thr Ala Lys Ser
Lys
  1               5                   10
15


<210> 14
<211> 15
<212> PRT
<213> Artificial Sequence

<220>
<223> Description of Artificial Sequence: peptides to
      generate polyclonal antibodies

<220>
<221> SITE
<222> (1)..(15)

<400> 14
Ile Asp Ile Asp Ile Ser Ser Arg Arg Arg Glu Asp Gln Ser
Leu
  1               5                   10
15


<210> 15
<211> 10

<212> PRT
<213> Artificial Sequence

<220>
<223> Description of Artificial Sequence: Myc-tag

<220>
<221> SITE
<222> (1)..(10)

<400> 15
Glu Gln Lys Leu Ile Ser Glu Glu Asp Leu
1               5                   10


<210> 16
<211> 981
<212> DNA
<213> Homo sapiens

<220>
<221> misc_feature
<222> (1)..(981)
<223> /note="Partial sequence of vector pMT2SM-AAP5"

<400> 16
ccaatggctg aattcatgga ctatacttca agtcagtgtg ggaaatatta
ttcatctgtg 60
ccagaggaag gaggggcaac ccatgtctat cgttatcaca gaggcgagtc
gaagctgcac 120
atgtgcttgg acatagggaa tggtcagaga aaagacagaa aaaagacatc
ccttggtcct 180
ggaggcagct atcaaatatc agagcatgct ccagaggcat cccagcctgc
tgagaacatc 240
tctaaggacc tctacataga agtatatcca gggacctatt ctgtcactgt
gggctcaaat 300
gacttaacca agaagactca tgtggtagca gttgattctg gacaaagcgt
ggacctggtc 360
ttccctgtgt gatgttgacc atcactgcca tcacatcacc ttttttaag
tagtaagaat 420
aaagccactg tatgattctc ttaatagcta tacattaatc ctgtttttag
tgctgactgg 480
gtcagccttc cgggaactgg agtctgtctc tttcagtgct tttttgtttg
tttggttggt 540
tgttttttga gacagtctcg ctctgttgcc caggctggag tgcagtggcg
tgatctcggc 600
tcaccgcaag ttccgcctcc cgggttcaca ccattctcct gcctcagcct
cccgagtagc 660
tggcactaca ggcacccgcc accatgcccg gctatttttt ttgtattttt
agtagagacg 720
gggtttcacc atgttggcca ggatggtctc gatctcttga cctcgtgatc
cacccacctt 780
ggcctcccaa agtgttggga ttacaggcgt gagccaccgc gcccggcctc
agtgcctttt 840
ttaacttgag ggtgtagagg tcctccacgc ttgtttgcct gaaagtaata
taatgatgct 900
gtctgaacag gttttactgc ttgctttcca agtaaaggtt aattatgata
ataaagagat 960

<210> 25
<211> 974
<212> DNA
<213> Homo sapiens

<220>
<223> Description of Combined DNA/RNA Molecule: sequence
      of full-length AAP-5

<220>
<221> CDS
<222> (91)..(795)
<223> /note="Sequence of full-length AAP5"

<400> 25
ctgcgccggc gccgccggga gcgctaggcc tggtccctct tcctaggata
gcgttgcgcg 60

catgcgcctt gacgagtgag ccggggagcc atg gac aac tgt ttg gcg
gcc gca  114
                                 Met Asp Asn Cys Leu Ala
Ala Ala
                                   1               5

gcg ctg aat ggg gtg gac cga cgt tcc ctg cag cgt tca gca
aag ctg   162
Ala Leu Asn Gly Val Asp Arg Arg Ser Leu Gln Arg Ser Ala
Lys Leu
     10                  15                  20

gct cta gaa gtg ctg gag agg gcc aag agg agg gcg gtg gac
tgg cat   210
Ala Leu Glu Val Leu Glu Arg Ala Lys Arg Arg Ala Val Asp
Trp His
 25                  30                  35
40

gcc ctg gag cgt ccc aaa ggc tgc atg ggg gtc ctt gcc cgg
gag gcg   258
Ala Leu Glu Arg Pro Lys Gly Cys Met Gly Val Leu Ala Arg
Glu Ala
                 45                  50
55

ccc cac cta gag aaa cag ccg gca gcc ggc ccg cag cgc gtt
ctc ccg   306
Pro His Leu Glu Lys Gln Pro Ala Ala Gly Pro Gln Arg Val
Leu Pro
             60                  65                  70

gga gag aga gaa gag aga ccc cca acc ctt agt gct tcc ttc
aga aca   354
Gly Glu Arg Glu Glu Arg Pro Pro Thr Leu Ser Ala Ser Phe
Arg Thr
         75                  80                  85

ttgggccttc gtggcctcga g
981

<210> 17
<211> 126
<212> PRT
<213> Homo sapiens

<220>
<221> SITE
<222> (1)..(126)
<223> /note="Amino-acid sequence of the analyzed region
      of AAP-5"

<400> 17
His Glu Gly Pro Met Ala Glu Phe Met Asp Tyr Thr Ser Ser
Gln Cys
  1               5                   10
15

Gly Lys Tyr Tyr Ser Ser Val Pro Glu Glu Gly Gly Ala Thr
His Val
             20                  25                  30

Tyr Arg Tyr His Arg Gly Glu Ser Lys Leu His Met Cys Leu
Asp Ile
         35                  40                  45

Gly Asn Gly Gln Arg Lys Asp Arg Lys Lys Thr Ser Leu Gly
Pro Gly
     50                  55                  60

Gly Ser Tyr Gln Ile Ser Glu His Ala Pro Glu Ala Ser Gln
Pro Ala
 65                  70                  75
80

Glu Asn Ile Ser Lys Asp Leu Tyr Ile Glu Val Tyr Pro Gly
Thr Tyr
                 85                  90
95

Ser Val Thr Val Gly Ser Asn Asp Leu Thr Lys Lys Thr His
Val Val
            100                 105                 110

Ala Val Asp Ser Gly Gln Ser Val Asp Leu Val Phe Pro Val
        115                 120                 125

<210> 18
<211> 123
<212> PRT
<213> Homo sapiens

<220>
<221> SITE

<222> (1)..(123)
<223> /note="Partial amino acid sequence of AAP-5"

<400> 18
Pro Met Ala Glu Phe Met Asp Tyr Thr Ser Ser Gln Cys Gly
Lys Tyr
1               5                   10
15

Tyr Ser Ser Val Pro Glu Glu Gly Gly Ala Thr His Val Tyr
Arg Tyr
            20                  25                  30

His Arg Gly Glu Ser Lys Leu His Met Cys Leu Asp Ile Gly
Asn Gly
        35                  40                  45

Gln Arg Lys Asp Arg Lys Lys Thr Ser Leu Gly Pro Gly Gly
Ser Tyr
    50                  55                  60

Gln Ile Ser Glu His Ala Pro Glu Ala Ser Gln Pro Ala Glu
Asn Ile
65                  70                  75
80

Ser Lys Asp Leu Tyr Ile Glu Val Tyr Pro Gly Thr Tyr Ser
Val Thr
                85                  90
95

Val Gly Ser Asn Asp Leu Thr Lys Lys Thr His Val Val Ala
Val Asp
            100                 105                 110

Ser Gly Gln Ser Val Asp Leu Val Phe Pro Val
        115                 120


<210> 19
<211> 108
<212> PRT
<213> Homo sapiens

<220>
<221> SITE
<222> (1)..(108)
<223> /note="BRCA-binding protein BRIP-1"

<400> 19
Gly Thr Ser Ser Cys Arg Arg Val Arg Ala Cys Gly Arg Ile
His His
1               5                   10
15

Asn Met Ala Asn Leu Phe Ile Arg Lys Met Val Asn Pro Leu
Leu Tyr
            20                  25                  30

Leu Ser Arg His Thr Val Lys Pro Arg Ala Leu Ser Thr Phe
Leu Phe
        35                  40                  45

Gly Ser Ile Arg Ser Ala Ala Pro Val Ala Val Glu Pro Gly
Ala Ala
    50                  55                  60

Val Arg Ser Leu Leu Ser Pro Gly Leu Leu Pro His Leu Leu
Pro Ala
 65                 70                  75
80

Leu Gly Phe Lys Asn Lys Thr Val Leu Lys Lys Arg Cys Lys
Asp Cys
                85                  90
95

Tyr Leu Val Lys Arg Arg Gly Arg Trp Tyr Val Tyr
            100                 105

<210> 20
<211> 651
<212> DNA
<213> Homo sapiens

<220>
<221> misc_feature
<222> (1)..(651)
<223> /note="Partial sequence of vector pMT2SM-AAP3"

<400> 20
ccgatggtag gcggcggcgg ggtcggcggc ggcctcctgg agaatgccaa
ccccctcatc 60
taccagcgct ctggggagcg gcctgtgacg gcaggcgagg aggacgagca
ggttcccgac 120
agcatcgacg cacgcgagat cttcgarctg attcgctcca tcaatgaccc
ggagcatcca 180
ctgacgctag aggagttgaa cgtagtagag caggtgcggg ttcaggttag
cgaccccgag 240
agtacagtgg ctgtggcttt cacaccaacc attccgcact gcagcatggc
caccettatt 300
ggtctgtcca tcaaggtcaa gcttctgcgc tcccttcctc agcgtttcaa
gatggacgtg 360
cacattactc cggggaccca tgcctcagag catgcagtga acaagcaact
tgcagataag 420
gagcgggtgg cagctgccct ggagaacacc cacctcttgg aggttgtgaa
tcagtgcctg 480
tcagcccgct cctgagcctg gcctttgacc cctcaacctg catactgggt
atcctggtcc 540
caactcctgc caagggctgt taccgttgtt ttcctggaat cactcacaaa
tgagaaacta 600
acatttgcct ttttgtaata aagttaattt atattcaaaa aaaaaaaaaa c
651

<210> 21
<211> 167
<212> PRT
<213> Homo sapiens

<220>
<221> SITE
<222> (1)..(167)
<223> /note="Amino acid sequence of the analyzed region
      of the Apoptin-associating clone AAP3"

<400> 21
His Glu Gly Pro Met Val Gly Gly Gly Gly Val Gly Gly Gly
Leu Leu
 1               5                   10
15

Glu Asn Ala Asn Pro Leu Ile Tyr Gln Arg Ser Gly Glu Arg
Pro Val
            20                  25                  30

Thr Ala Gly Glu Glu Asp Glu Gln Val Pro Asp Ser Ile Asp
Ala Arg
        35                  40                  45

Glu Ile Phe Asp Leu Ile Arg Ser Ile Asn Asp Pro Glu His
Pro Leu
    50                  55                  60

Thr Leu Glu Glu Leu Asn Val Val Glu Gln Val Arg Val Gln
Val Ser
65                  70                  75
80

Asp Pro Glu Ser Thr Val Ala Val Ala Phe Thr Pro Thr Ile
Pro His
                85                  90
95

Cys Ser Met Ala Thr Leu Ile Gly Leu Ser Ile Lys Val Lys
Leu Leu
            100                 105                 110

Arg Ser Leu Pro Gln Arg Phe Lys Met Asp Val His Ile Thr
Pro Gly
        115                 120                 125

Thr His Ala Ser Glu His Ala Val Asn Lys Gln Leu Ala Asp
Lys Glu
    130                 135                 140

Arg Val Ala Ala Ala Leu Glu Asn Thr His Leu Leu Glu Val
Val Asn
145                 150                 155
160

Gln Cys Leu Ser Ala Arg Ser
                165

<210> 22
<211> 770
<212> DNA
<213> Homo sapiens

<220>
<223> Description of Combined DNA/RNA Molecule: sequence
      of AAP-4

<220>
<221> CDS
<222> (12)..(512)
<223> /note="Partial sequence of vector pMT2SM-AAP4"

<400> 22
gccacgaagg c cgg gag agc tcg ccc tgc acc tac ata act cgg
cgg tca  50
             Arg Glu Ser Ser Pro Cys Thr Tyr Ile Thr Arg
Arg Ser
               1               5                   10

gtg agg aca aga aca aat ctg aag gag gcc tct gac atc aag
ctt gaa   98
Val Arg Thr Arg Thr Asn Leu Lys Glu Ala Ser Asp Ile Lys
Leu Glu
     15                  20                  25

cca aat acg ttg aat ggc tat aaa agc agt gtg acg gaa cct
tgc ccc   146
Pro Asn Thr Leu Asn Gly Tyr Lys Ser Ser Val Thr Glu Pro
Cys Pro
 30                  35                  40
45

gac agt ggt gaa cag ctg cag cca gct cct gtg ctg cag gag
gaa gaa   194
Asp Ser Gly Glu Gln Leu Gln Pro Ala Pro Val Leu Gln Glu
Glu Glu
                    50                  55
60

ctg gct cat gag act gca caa aaa ggg gag gca aag tgt cat
aag agt   242
Leu Ala His Glu Thr Ala Gln Lys Gly Glu Ala Lys Cys His
Lys Ser
            65                  70                  75

gac aca ggc atg tcc aaa aag aag tca cga caa gga aaa ctt
gtg aaa   290
Asp Thr Gly Met Ser Lys Lys Lys Ser Arg Gln Gly Lys Leu
Val Lys
        80                  85                  90

cag ttt gca aaa ata gag gaa tct act cca gtg cac gat tct
cct gga   338

Gln Phe Ala Lys Ile Glu Glu Ser Thr Pro Val His Asp Ser
Pro Gly
     95                  100                 105

aaa gac gac gcg gta cca gat ttg atg ggt ccc cat tct gac
cag ggt   386
Lys Asp Asp Ala Val Pro Asp Leu Met Gly Pro His Ser Asp
Gln Gly
110                 115                 120
125

gag cac agt ggc act gtg ggc gtg cct gtg agc tac aca gac
tgt gct   434
Glu His Ser Gly Thr Val Gly Val Pro Val Ser Tyr Thr Asp
Cys Ala
                130                 135
140

cct tca ccc gtc ggt tgt tca gtt gtg aca tca gat agc ttc
aaa aca   482
Pro Ser Pro Val Gly Cys Ser Val Val Thr Ser Asp Ser Phe
Lys Thr
            145                 150                 155

aaa gac agc ttt aga act gca aaa aag taa aaagaagagg
cgaatcacaa     532
Lys Asp Ser Phe Arg Thr Ala Lys Lys
        160                 165

ggtatgatgc acagttaatc ctagaaaata actctgggag tcccaaattg
actcttcgta 592

ggcgtcatga tagcagcagc aaaacaaatg gaccaagaga atgatgggaa
tgaaactctt 652

cccaaaatta agcatcaagt ttaagccaaa gaccatgaca acgataacaa
tctcgatgta 712

gcaaagttat aagggtttag ctcaggatta ggaatgtttc acaaaattaa
aaaggcat   770


<210> 23
<211> 166
<212> PRT
<213> Homo sapiens
<223> Description of Combined DNA/RNA Molecule: sequence
      of AAP-4

<400> 23
Arg Glu Ser Ser Pro Cys Thr Tyr Ile Thr Arg Arg Ser Val
Arg Thr
  1               5                  10
15
Arg Thr Asn Leu Lys Glu Ala Ser Asp Ile Lys Leu Glu Pro
Asn Thr
             20                  25                  30

Leu Asn Gly Tyr Lys Ser Ser Val Thr Glu Pro Cys Pro Asp
Ser Gly
        35                  40                  45
Glu Gln Leu Gln Pro Ala Pro Val Leu Gln Glu Glu Glu Leu
Ala His
    50                  55                  60
Glu Thr Ala Gln Lys Gly Glu Ala Lys Cys His Lys Ser Asp
Thr Gly
 65                 70                  75
80
Met Ser Lys Lys Lys Ser Arg Gln Gly Lys Leu Val Lys Gln
Phe Ala
                85                  90
95
Lys Ile Glu Glu Ser Thr Pro Val His Asp Ser Pro Gly Lys
Asp Asp
            100                 105                 110
Ala Val Pro Asp Leu Met Gly Pro His Ser Asp Gln Gly Glu
His Ser
        115                 120                 125
Gly Thr Val Gly Val Pro Val Ser Tyr Thr Asp Cys Ala Pro
Ser Pro
    130                 135                 140
Val Gly Cys Ser Val Val Thr Ser Asp Ser Phe Lys Thr Lys
Asp Ser
145                 150                 155
160
Phe Arg Thr Ala Lys Lys
                165

<210> 24
<211> 256
<212> PRT
<213> Homo sapiens

<220>
<221> SITE
<222> (1)..(256)
<223> /note=Amino acid sequence of the analyzed region
      of the Apoptin-associating clone AAP4, wherein X
      is a stopcodon"

<400> 24
His Glu Gly Arg Glu Ser Ser Pro Cys Thr Tyr Ile Thr Arg
Arg Ser
  1               5                  10
15

Val Arg Thr Arg Thr Asn Leu Lys Glu Ala Ser Asp Ile Lys
Leu Glu
            20                  25                  30

Pro Asn Thr Leu Asn Gly Tyr Lys Ser Ser Val Thr Glu Pro
Cys Pro
        35                  40                  45

Asp Ser Gly Glu Gln Leu Gln Pro Ala Pro Val Leu Gln Glu
Glu Glu
    50                  55                  60

Leu Ala His Glu Thr Ala Gln Lys Gly Glu Ala Lys Cys His
Lys Ser
65                  70                  75
80

Asp Thr Gly Met Ser Lys Lys Lys Ser Arg Gln Gly Lys Leu
Val Lys
                85                  90
95

Gln Phe Ala Lys Ile Glu Glu Ser Thr Pro Val His Asp Ser
Pro Gly
            100                 105                 110

Lys Asp Asp Ala Val Pro Asp Leu Met Gly Pro His Ser Asp
Gln Gly
        115                 120                 125

Glu His Ser Gly Thr Val Gly Val Pro Val Ser Tyr Thr Asp
Cys Ala
    130                 135                 140

Pro Ser Pro Val Gly Cys Ser Val Val Thr Ser Asp Ser Phe
Lys Thr
145                 150                 155
160

Lys Asp Ser Phe Arg Thr Ala Lys Lys Xaa Lys Glu Glu Ala
Asn His
                165                 170
175

Lys Val Xaa Cys Thr Val Asn Pro Arg Lys Xaa Leu Trp Glu
Ser Gln
            180                 185                 190

Ile Asp Ser Ser Xaa Ala Ser Xaa Xaa Gln Gln Gln Asn Lys
Trp Thr
        195                 200                 205

Lys Arg Met Met Gly Met Lys Leu Phe Pro Lys Leu Ser Ile
Lys Phe
    210                 215                 220

Lys Pro Lys Thr Met Thr Thr Ile Thr Ile Ser Met Xaa Gln
Ser Tyr
225                 230                 235
240

Lys Gly Leu Ala Gln Asp Xaa Glu Cys Phe Thr Lys Leu Lys
Arg His
                245                 250
255

atg gct gaa ttc atg gac tat act tca agt cag tgt ggg aaa
tat tat   402
Met Ala Glu Phe Met Asp Tyr Thr Ser Ser Gln Cys Gly Lys
Tyr Tyr
 90                  95                  100

tca tct gtg cca gag gaa gga ggg gca acc cat gtc tat cgt
tat cac   450
Ser Ser Val Pro Glu Glu Gly Gly Ala Thr His Val Tyr Arg
Tyr His
105                 110                 115
120

aga ggc gag tcg aag ctg cac atg tgc ttg gac ata ggg aat
ggt cag   498
Arg Gly Glu Ser Lys Leu His Met Cys Leu Asp Ile Gly Asn
Gly Gln
                125                 130
135

aga aaa gac aga aaa aag aca tcc ctt ggt cct gga ggc agc
tat caa   546
Arg Lys Asp Arg Lys Lys Thr Ser Leu Gly Pro Gly Gly Ser
Tyr Gln
            140                 145                 150

ata tca gag cat gct cca gag gca tcc cag cct gct gag aac
atc tct   594
Ile Ser Glu His Ala Pro Glu Ala Ser Gln Pro Ala Glu Asn
Ile Ser
        155                 160                 165

aag gac ctc tac ata gaa gta tat cca ggg acc tat tct gtc
act gtg   642
Lys Asp Leu Tyr Ile Glu Val Tyr Pro Gly Thr Tyr Ser Val
Thr Val
    170                 175                 180

ggc tca aat gac tta acc aag aag act cat gtg gta gca gtt
gat tct   690
Gly Ser Asn Asp Leu Thr Lys Lys Thr His Val Val Ala Val
Asp Ser
185                 190                 195
200

gga caa agc gtg gac ctg gtc ttc cct gtg tga tgt tga cca
tca ctg   738
Gly Gln Ser Val Asp Leu Val Phe Pro Val     Cys     Pro
Ser Leu
                205                 210
215

cca tca cat cac ctt ttt tta agt agt aag aat aaa gcc act
gta tga   786
Pro Ser His His Leu Phe Leu Ser Ser Lys Asn Lys Ala Thr
Val
            220                 225                 230

ttc tct taa tagctataca ttaatcctgt ttttagtgct gactgggtca
835
Phe Ser
        235

gccttccggg aactggagtc tgtctctttc agtgcttttt tgtttgtttg
gttggttttt 895

ttttgagaca gtctcactct gttgcccagg ctggagtgca gtggcgtgat
ctcggctcac 955

tgcaagttcc gcctcccgg
974



<210> 26
<211> 210
<212> PRT
<213> Homo sapiens
<223> Description of Combined DNA/RNA Molecule: sequence
      of full-length AAP-5

<400> 26
Met Asp Asn Cys Leu Ala Ala Ala Ala Leu Asn Gly Val Asp
Arg Arg
  1               5                   10
15
Ser Leu Gln Arg Ser Ala Lys Leu Ala Leu Glu Val Leu Glu
Arg Ala
             20                  25                  30
Lys Arg Arg Ala Val Asp Trp His Ala Leu Glu Arg Pro Lys
Gly Cys
         35                  40                  45
Met Gly Val Leu Ala Arg Glu Ala Pro His Leu Glu Lys Gln
Pro Ala
     50                  55                  60
Ala Gly Pro Gln Arg Val Leu Pro Gly Glu Arg Glu Glu Arg
Pro Pro
 65                  70                  75
80
Thr Leu Ser Ala Ser Phe Arg Thr Met Ala Glu Phe Met Asp
Tyr Thr
                 85                  90
95
Ser Ser Gln Cys Gly Lys Tyr Tyr Ser Ser Val Pro Glu Glu
Gly Gly
            100                 105                 110
Ala Thr His Val Tyr Arg Tyr His Arg Gly Glu Ser Lys Leu
His Met
        115                 120                 125
Cys Leu Asp Ile Gly Asn Gly Gln Arg Lys Asp Arg Lys Lys
Thr Ser
    130                 135                 140
Leu Gly Pro Gly Gly Ser Tyr Gln Ile Ser Glu His Ala Pro
Glu Ala
145                 150                 155
160

Ser Gln Pro Ala Glu Asn Ile Ser Lys Asp Leu Tyr Ile Glu
Val Tyr
                165                 170
175
Pro Gly Thr Tyr Ser Val Thr Val Gly Ser Asn Asp Leu Thr
Lys Lys
            180                 185                 190
Thr His Val Val Ala Val Asp Ser Gly Gln Ser Val Asp Leu
Val Phe
        195                 200                 205
Pro Val
    210



<210> 27
<211> 18
<212> PRT
<213> Homo sapiens
<223> Description of Combined DNA/RNA Molecule: sequence
      of full-length AAP-5

<400> 27
Pro Ser Leu Pro Ser His His Leu Phe Leu Ser Ser Lys Asn
Lys Ala
  1               5                  10
15
Thr Val

## Description

The invention relates to the field of apoptosis. Apoptosis is an active and programmed physiological process for eliminating superfluous, altered or malignant cells (Earnshaw, 1995, Duke et al., 1996). Apoptosis is characterized by shrinkage of cells, segmentation of the nucleus, condensation and cleavage of DNA into domain-sized fragments, in most cells followed by internucleosomal degradation. The apoptotic cells fragment into membrane-enclosed apoptotic bodies. Finally, neighbouring cells and/or macrophages will rapidly phagocytose these dying cells (Wyllie et al., 1980, White, 1996). Cells grown under tissue-culture conditions and cells from tissue material can be analysed for being apoptotic with agents staining DNA, as e.g. DAPI, which stains normal DNA strongly and regularly, whereas apoptotic DNA is stained weakly and/or irregularly (Noteborn et al., 1994, Telford et al., 1992).

The apoptotic process can be initiated by a variety of regulatory stimuli (Wyllie, 1995, White 1996, Levine, 1997). Changes in the cell survival rate play an important role in human pathogenesis of diseases, e.g. in cancer development and auto-immune diseases, where enhanced proliferation or decreased cell death (Kerr et al., 1994, Paulovich, 1997) is observed. A variety of chemotherapeutic compounds and radiation have been demonstrated to induce apoptosis in tumor cells, in many instances via wild-type p53 protein (Thompson, 1995, Bellamy et al., 1995, Steller, 1995, McDonell et al., 1995).

Many tumors, however, acquire a mutation in p53 during their development, often correlating with poor response to cancer therapy. Certain transforming genes of tumorigenic DNA viruses can inactivate p53 by directly binding to it (Teodoro, 1997). An example of such an agent is the large T antigen of the tumor DNA virus SV40. For several (leukemic) tumors, a high expression level of the proto-oncogene Bcl-2 or Bcr-abl is associated with a strong resistance to various apoptosis-inducing chemotherapeutic agents (Hockenberry 1994, Sachs and Lotem, 1997).

For such tumors lacking functional p53 (representing more than half of the tumors) alternative anti-tumor therapies are under development based on induction of apoptosis independent of p53 (Thompson 1995, Paulovich et al., 1997). One has to search for the factors involved in induction of apoptosis, which do not need p53 and/or cannot be blocked by anti-apoptotic activities, such as Bcl-2 or Bcr-abl-like ones. These factors might be part of a distinct apoptosis pathway or might be (far) downstream of the apoptosis inhibiting compounds.

Apoptin is a small protein derived from chicken anemia virus (CAV; Noteborn and De Boer, 1995, Noteborn et al., 1991, Noteborn et al., 1994; 1998a), which can induce apoptosis in human malignant and transformed cell lines, but not in untransformed human cell cultures. *In vitro,* Apoptin fails to induce programmed cell death in normal lymphoid, dermal, epidermal, endothelial and smooth-muscle cells. However, when normal cells are transformed they become susceptible to apoptosis by Apoptin. Long-term expression of Apoptin in normal human fibroblasts revealed that Apoptin has no toxic or transforming activity in these cells (Danen-van Oorschot, 1997 and Noteborn, 1996).

In normal cells, Apoptin was found predominantly in the cytoplasm, whereas in transformed or malignant cells i.e. characterized by hyperplasia, metaplasia or dysplasia, it was located in the nucleus, suggesting that the localization of Apoptin is related to its activity (Danen-van Oorschot et al. 1997).
Apoptin-induced apoptosis occurs in the absence of functional p53 (Zhuang et al., 1995a), and cannot be blocked by Bcl-2, Bcr-abl (Zhuang et al., 1995), or the Bcl-2-associating protein BAG-1 (Danen-Van Oorschot, 1997a, Noteborn, 1996).

Therefore, Apoptin is a therapeutic compound for the selective destruction of tumor cells, or other hyperplasia, metaplasia or dysplasia, especially for those tumor cells which have become resistant to (chemo)-therapeutic induction of apoptosis, due to the lack of functional p53 and (over)-expression of Bcl-2 and other apoptosis-inhibiting agents (Noteborn and Pietersen, 1998). It appears, that even pre-malignant, minimally transformed cells, are sensitive to the death-inducing effect of Apoptin. In addition, Noteborn and Zhang (1998) have shown that Apoptin-induced apoptosis can be used as diagnosis of cancer-prone cells and treatment of cancer-prone cells.

The fact that Apoptin does not induce apoptosis in normal human cells, at least not *in vitro,* shows that a toxic effect of Apoptin treatment *in vivo* will be very low. Noteborn and Pietersen (1998) and Pietersen et al. (1999) have provided evidence that adenovirus expressed Apoptin does not have an acute toxic effect *in vivo.* In addition, in nude mice it was shown that Apoptin has a strong anti-tumor activity.

However, to further enlarge the array of therapeutic anti-cancer or anti-auto-immune-disease compounds available in the art, additional therapeutic compounds are desired that are designed to work alone, sequentially to, or jointly with Apoptin, especially in those cases wherein p53 is (partly) non-functional.

The invention provides novel therapeutic possibilities, for example novel combinatorial therapies or novel therapeutic compounds that can work alone, sequentially to, or jointly with Apoptin, especially in those cases wherein p53 is (partly) non-functional.

In a first embodiment, the invention provides an isolated or recombinant nucleic acid or functional equivalent or fragment thereof encoding an Apoptin-associating proteinaceous substance capable of providing apoptosis, alone or in combination with other apoptosis inducing substances, such as Apoptin. Proteinaceous substance herein is defined as a substance comprising a peptide, polypeptide or protein, optionally having been modified by for example glycosylation, myristilation, phosphorylation, the addition of lipids, by homologous or heterologous di- or multi-merisation, or any other (posttranslational) modifications known in the art.

Apoptin-associating herein is defined as belonging to the cascade of substances specifically involved in the cascade of events found in the apoptosis pathway as inducible by Apoptin, preferably those substances that are specifically involved in the p53-independent apoptosis pathway.

In a preferred embodiment, the invention provides an isolated or recombinant nucleic acid or functional equivalent or fragment thereof encoding an Apoptin-associating proteinaceous substance capable of providing apoptosis derived from a cDNA library, preferably a vertebrate cDNA library, such as derivable from poultry, but more preferably a mammalian cDNA library, preferably wherein said cDNA library comprises human cDNA. An Apoptin-associating proteinaceous substance obtained by determining a vertebrate homologue (preferably human) of an Apoptin-associating proteinaceous substance derived from an invertebrate cDNA library is also included.

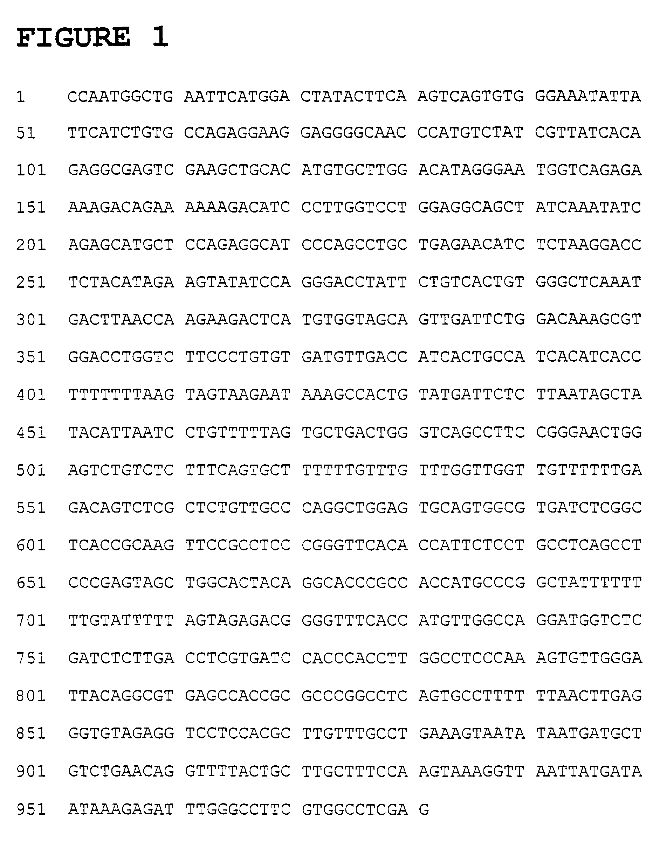

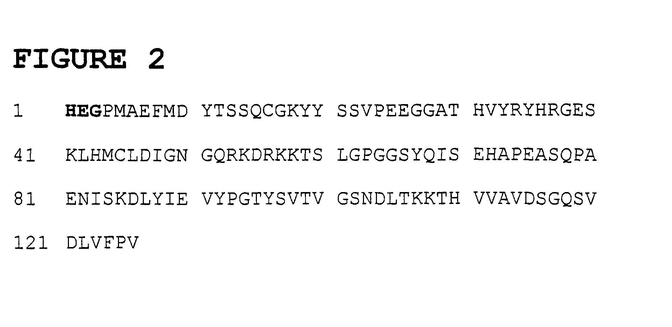

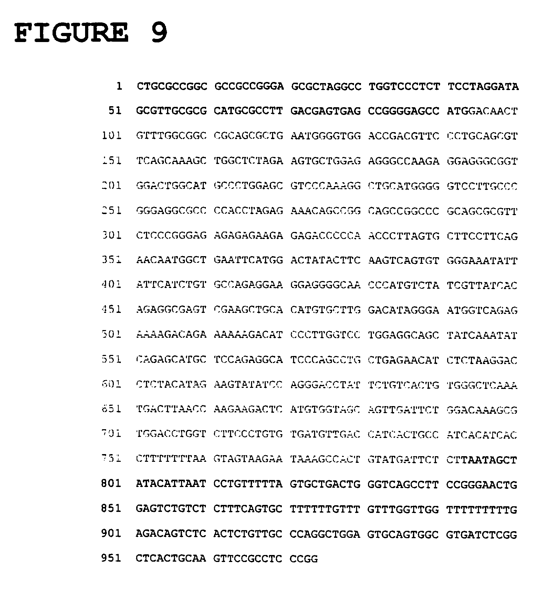

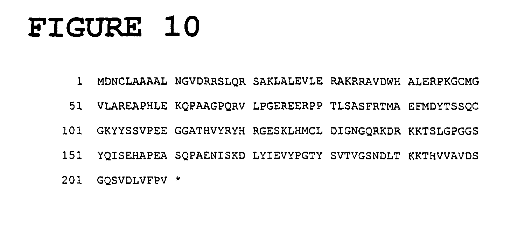

In another embodiment, the invention provides an isolated or recombinant nucleic acid or functional equivalent or fragment thereof encoding an Apoptin-associating proteinaceous substance capable of providing apoptosis capable of hybridising to a nucleic acid molecule encoding an Apoptin-associating proteinaceous substance capable of providing apoptosis as shown in figure 1 and/or 9, in particular encoding a novel protein capable of providing apoptosis or functional equivalent or functional fragment thereof called Apoptin-associating protein 5, abbreviated herein also as AAP-5. Figure 1 shows a fragment of the complete AAP-5 fragment as depicted in Figure 9. Both nucleotide sequences encode a protein (Figure 2 and Figure 10) with at least the capability of binding to Apoptin and providing apoptosis (as disclosed herein within the experimental part). Of course, an isolated or recombinant nucleic acid or functional equivalent or fragment thereof encoding an additional Apoptin-associating proteinaceous substance capable of associating with the partial or full-length AAP-5 protein is herewith also provided, means and methods to arrive at such an additional protein located in the Apoptin cascade follow those of the detailed description given herein. Knowledge derived from studying the partial or full-length AAP-5 is exploited to determine a functional pathway in which partial or full-length AAP-5 is involved, thus allowing the design of further therapeutic means of intervening in such a pathway.

In particular, the invention provides an isolated or recombinant nucleic acid or functional equivalent or fragment thereof encoding an Apoptin-associating proteinaceous substance capable of providing apoptosis being at least 60% homologous, preferably at least 70%, more preferably at least 80%, even more preferably at least 90% and most preferably at least 95% homologous to a nucleic acid molecule, or to a functional equivalent or functional fragment thereof, encoding an Apoptin-associating proteinaceous substance as shown in figure 1 or 9.

Furthermore, the invention provides a vector comprising a nucleic acid according to the invention. Examples of such a vector are given in the detailed description given herein; such as vector pMT2SM-AAP-5, pMT2SM vector expressing Myc-tagged AAP-5 cDNA, a plasmid expressing an Apoptin-associating protein fragment, and so on. This and other vectors are for example useful in finding additional Apoptin-associating proteinaceous substances from the cascade, as defined above.

In yet another embodiment, the invention provides a vector comprising a nucleic acid according to the invention, said vector comprising a gene-delivery vehicle, making the invention very useful in gene therapy. By equipping a gene delivery vehicle with a nucleic acid according to the invention, and by targeting said vehicle to a cell or cells that have been over-proliferating and/or have shown decreased death rates, said gene delivery vehicle provides said cell or cells with the necessary means for apoptosis, providing far reaching therapeutic possibilities.

Furthermore, the invention provides a host cell comprising a nucleic acid or a vector according to the invention. Examples comprise transduced bacterial or yeast cells as described in the detailed description herein. Preferred is a host cell according to the invention which is a transduced eukaryotic cell such as a yeast cell or a vertebrate cell, such as mammalian or Cos cells transduced with a nucleic acid or vector according to the invention. Said cells are in general capable to express or produce a proteinaceous substance capable of providing apoptosis with the ability to associate with Apoptin.

The invention furthermore provides an isolated or recombinant Apoptin-associating proteinaceous substance capable of providing apoptosis. As for example shown herein in figures 2 and 10, expression of such Apoptin-associating proteinaceous substance in cells, such as tumour cells, or other over-proliferating cells, induces the apoptotic process. It can do so alone, or in the presence of other apoptosis inducing substances such as Apoptin, and especially so independent of p53, showing that also in those cases where (functional) p53 is absent apoptosis can be induced by a substance according to the invention. In particular, the invention provides a proteinaceous substance according to the invention encoded by a nucleic acid according to the invention, for example comprising at least a part of an amino acid sequence as shown in figure 2 or 10 (AAP-5) or a functional equivalent or functional fragment thereof capable of providing apoptosis alone or in combination with other apoptosis inducing substances such as Apoptin.

The invention also provides an isolated or synthetic antibody specifically recognising a proteinaceous substance or functional equivalent or functional fragment thereof according to the invention. Such an antibody is for example obtainable by immunising an experimental animal with an Apoptin-associating proteinaceous substance or an immunogenic fragment or equivalent thereof and harvesting polyclonal antibodies from said immunised animal, or obtainable by other methods known in the art such as by producing monoclonal antibodies, or (single chain) antibodies or binding proteins expressed from recombinant nucleic acid derived from a nucleic acid library, for example obtainable via phage display techniques. Examples of such an antibody are given in the detailed description herein.

With such an antibody, the invention also provides a proteinaceous substance specifically recognisable by such an antibody according to the invention, for example obtainable via immunoprecipitation, Western Blotting, or other immunological techniques known in the art.

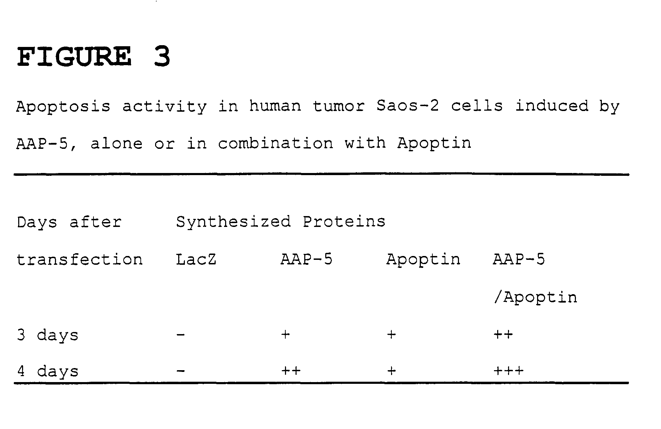

Furthermore, the invention provides use of a nucleic acid, vector, host cell, or proteinaceous substance according to the invention for the induction of tumor-specific apoptosis, as for example shown in figure 3. In particular, such use is provided wherein said apoptosis is p53-independent. In particular, such use is also provided further comprising use of a nucleic acid encoding Apoptin or a functional equivalent or fragment thereof or use of Apoptin or a functional equivalent or fragment thereof. As can be seen from figure 3, combining these Apoptin-inducing substances increases the percentage apoptosis of treated tumour cells.

Such use as provided by the invention is particularly useful from a therapeutic viewpoint. The invention provides herewith a pharmaceutical composition comprising a nucleic acid, vector, host cell, or proteinaceous substance according to the invention. In addition, such a pharmaceutical composition according to the invention is provided further comprising a nucleic acid encoding Apoptin or a functional equivalent or fragment thereof.

Such a pharmaceutical composition is in particular provided for the induction of apoptosis, for example wherein said apoptosis is p53-independent, for the treatment of a disease where enhanced cell proliferation or decreased cell death is observed, as is in general the case when said disease comprises cancer or auto-immune disease. Herewith the invention provides a method for treating an individual carrying a disease where enhanced cell proliferation or decreased cell death is observed comprising treating said individual with a pharmaceutical composition according to the invention. In particular these compositions comprise a factor of an apoptosis pathway, which is specific for transformed cells. Therefore, these compositions are essential for new treatments, but also for diagnosis of diseases related with aberrances in the apoptotic process, such as cancer, cancer-proneness and auto-immune diseases.

The invention will be explained in more detail in the following detailed description, which is not limiting the invention.

### Detailed description

We have used the yeast-2 hybrid system (Durfee et al., 1993) to identify Apoptin-associating cellular compounds, which are essential in the induction of apoptosis. The used system is an *in vivo* strategy to identify human proteins capable of physically associating with Apoptin. It has been used to screen cDNA libraries for clones encoding proteins capable of binding to a protein of interest (Fields and Song, 1989, Yang et al., 1992). The invention provides for example a novel Apoptin-associating protein, which is named Apoptin-associating protein 5 abbreviated as AAP-5.. The invention also provides a method for inducing apoptosis through interference with the function of this newly discovered AAP-5 protein or functional equivalents or fragments thereof and/or the induction of apoptosis by means of (over)expression of AAP-5 or related gene or functional equivalents or fragments thereof.

The invention also provides an anti-tumor therapy based on the interference with the function of AAP-5-like proteins and/or its (over)expression. The presence of AAP-5-like protein can result in the induction of the opposite process of cell transformation, namely apoptosis. The invention furthermore provides the mediator of Apoptin-induced apoptosis. The invention provides a therapy for cancer, autoimmune diseases or related diseases which is based on AAP-5-like proteins alone or in combination with Apoptin and/or Apoptin-like compounds.

### Construction of pGBT9-VP3

For the construction of the bait plasmid, which enables the identification of Apoptin-associating proteins by means of a yeast-two-hybrid system, plasmid pET-16b-VP3 (Noteborn, unpublished results) was treated with *Nde*I and *Bam*HI. The 0.4 kb *Nde*I*-Bam*HI DNA fragment was isolated from low-melting-point agarose. Plasmid pGBT9 (Clontech Laboratories, Inc, Palo Alto, USA) was treated with the restriction enzymes *Eco*RI and *Bam*HI. The about 5.4-kb DNA fragment was isolated and ligated to an *Eco*RI*-Nde*I linker and the 0.4-kb DNA fragment containing the Apoptin-encoding sequences starting from its own ATG-initiation codon. The final construct containing a fusion gene of the GAL4-binding domain sequence and Apoptin under the regulation of the yeast promoter ADH was called pGBT-VP3 and was proven to be correct by restriction-enzyme analysis and DNA-sequencing according to the Sanger method (1977).

All cloning steps were essentially carried out as described by Maniatis et al. (1992). The plasmid pGBT-VP3 was purified by centrifugation in a CsCl gradient and column chromatography in Sephacryl S500 (Pharmacia).

### GAL4-activation domain-tagged cDNA library

The expression vector pACT, containing the cDNAs from Epstein-Barr-virus-transformed human B cells fused to the GAL4 transcriptional activation domain, was used for detecting Apoptin-associating proteins. The pACT c-DNA library is derived from the lambda-ACT cDNA library, as described by Durfee et al. 1993.

### Bacterial and Yeast strains

The E.coli strain JM109 was the transformation recipient for the plasmid pGBT9 and pGBT-VP3. The bacterial strain electromax/DH10B was used for the transformation needed for the recovery of the Apoptin-associating pACT-cDNAs, and was obtained from GIBCO-BRL, USA.

The yeast strain Y190 was used for screening the cDNA library, and all other transformations, which are part of the used yeast-two-hybrid system.

### Media

For drug selections Luria Broth (LB) plates for E.coli were supplemented with ampicillin (50 microgram per ml). Yeast YPD and SC media were prepared as described by Rose et al. (1990).

### Transformation of competent yeast strain Y190 with plasmids pGBT-VP3 and pACT-cDNA and screening for beta-galactosidase activity.

The yeast strain Y190 was made competent and transformed according to the methods described by Klebe et al. (1983). The yeast cells were first transformed with pGBT-VP3 and subsequently transformed with pACT-cDNA, and these transformed yeast cells were grown on histidine-minus plates, also lacking leucine and tryptophan.

Hybond-N filters were layed on yeast colonies, which were histidine-positive and allowed to wet completely. The filters were lifted and submerged in liquid nitrogen to permeabilize the yeast cells. The filters were thawed and layed with the colony side up on Whattman 3MM paper in a petridish with Z-buffer (Per liter: 16.1 gr Na₂HPO₄.7H₂O, 5.5 gr NaH₂PO₄.H₂O, 0.75 gr KCl and 0,246 gr MgSO₄.7H₂O, pH 7.0) containing 0.27% beta-mercapto-ethanol and 1 mg/ml X-gal. The filters were incubated for at least 15 minutes or during night.

### Recovery of plasmids from yeast

Total DNA from yeast cells, which were histidine- and beta-galactosidase-positive, was prepared by using the glusulase-alkaline lysis method as described by Hoffman and Winston (1987) and used to transform Electromax/DH10B bacteria via electroporation using a Bio-Rad GenePulser according the manufacturer's specifications.

Transformants were plated on LB media containing the antibiotic agent ampicillin.

### Isolation of Apoptin-associating pACT clones

By means of colony-filter assay the colonies were lysed and hybridized to a radioactive-labeled 17-mer oligomer, which is specific for pACT (see also section Sequence analysis). Plasmid DNA was isolated from the pACT-clones, and by means of *Xho*I digestion analysed for the presence of a cDNA insert.

### Sequence analysis

The subclone containing the sequence encoding Apoptin-associating protein was partially sequenced using dideoxy NTPs according to the Sanger-method, which was performed by Eurogentec, Seraing, Belgium or BaseClear, Leiden, The Netherlands). The used sequencing primer was a pACT-specific 17-mer comprising of the DNA-sequence 5'-TACCACTACAATGGATG-3' or internal primers comprising the described novel AAP-5 sequences.

The sequences of the Apoptin-associating cDNAs were compared with known gene sequences from the EMBL/Genbank.

### Generation and testing of antibodies

In order to generate polyclonal antisera against the protein predicted to be encoded by the partial AAP-5 clone, Eurogentec (Belgium) designed three peptides predicted to be antigenic. These peptides were, for AAP-5: 1) Residues 27-41: CGGATHVYRYHRGESK; 2) Residues 49-63: GNGQRKDRKKTSLGPC; and 3) Residues 71-85: EHAPEASQPAENISKC. (Note: numbering same as in Figure 2, and underlined C residues are added for technical reasons and are not part of the actual AAP sequence). These peptides were synthesized at Eurogentec and all subsequent antibody syntheses were also performed there.

Briefly, the three peptides were coupled to KLH and injected as a cocktail into two separate specific pathogen free rabbits with an immunization schedule of one injection and three subsequent boosts. Blood samples were taken before and after immunization. The sera were tested in-house by ELISA for specific reactivity to the peptide cocktail. The titers from each rabbit were high. Furthermore, for certain subsequent purposes, the AAP-5 antibodies were immune-purified using peptide cocktail coupled to immobilized diaminodipropylamine agarose columns (Pierce) according to the manufacturer's protocol.

The AAP-5 antibody of the two generated with the highest affinity was selected for further use. We tested the efficacy of this antibody by transfecting 6 cm plates of sub-confluent primate COS-7 and human U2OS cells using the calcium phosphate co-precipitation method with 5 µg of the AAP-5-myc construct (partial clone), and as a control, untransfected cells. Two days post-transfection, cells were washed briefly in PBS, lysed in RIPA buffer (10 mM Tris 7.5, 150 mM NaCl, 0.1% SDS, 1.0% NP-49 and 1.0 % sodium deoxycholate), clarified by centrifugation, and the supernatant fractionated on SDS-denaturing polyacrylamide gel electrophoresis. Proteins were Western-transferred to PVDF membranes (Immobilon, Millipore) using standard methodology. Membranes were blocked in 5% non-fat dry milk in tris-buffered saline containing 0.1% Tween-20, then incubated in the unpurified AAP-5 antisera at a concentration of 1:5000. After a brief wash, membranes were further incubated in HRP-conjugated goat-anti-rabbit Ig at a concentration of 1:2000. After a thorough series of wash steps, proteins were detected using enhanced chemiluminescence (Amersham) according to the manufacturer's protocol, and exposed to x-ray film and developed using standard automated machinery. In addition, we tested the purified AAP-5 antibody using immunoprecipitation in a manner the same as above, except that after centrifugation, the supernatant was added to 10 µl of purified AAP-5 antibody pre-coupled to protein-A-sepharose beads, incubated for 1 hour with tumbling, then washed before fractionation of SDS-PAGE gels and Western analysis. Detection in this case was performed with the anti-myc tag monoclonal antibody 9E10 (Evan et al.). Finally, the purified AAP-5 antibody was tested for utility in immunofluorescence by including glass coverslips in the above transfections. Coverslips were fixed with 4% paraformaldehyde, blocked with normal goat serum, incubated in a concentration of 1:10, washed, incubated in a 1:100 dilution of conjugated goat-anti-rabbit Ig, mounted in DAPI/DABCO/glycerol and visualized with fluorescence microscopy.

### Northern analysis

To examine the RNA expression pattern of AAP-5, we tested commercially-manufactured Northern blots (Invitrogen, cat. 1999, #D2801-50) containing poly-A+ RNA from human fetal brain, liver, lung, muscle tissue and from adult lung. The DNA probe was derived from a *Xho*I restriction fragment encompassing the entire insert of the AAP-5-myc partial clone. This probe was labelled with ³²P-dATP using the MegaPrime kit of Amersham. All prehybridization, hybridization and washing steps were done according to the Northern blot manufacturer's recommendation. Blots were subjected to autoradiography and developed using standard automated methods.

### Cloning of full-length AAP-5

The complete ORF of AAP-5 was amplified from a human brain cDNA library (Clontech - Marathon Ready cDNA).
The RT-PCR was performed according to the manufacturer's instructions (Clontech - Advantage®2 PCR kit).

| RT-PCR | Sample volume 50 µl |
|---|---|
| Brain cDNA | 2 µl |
| 5'-primer (50 µM) | 1 µl |
| 3'-primer (50 µM) | 1 µl |
| 10x cDNA PCR reaction buffer | 5 µl |
| dNTP mix (10 mM) | 1 µl |
| 50x Advantage 2 Polymerase mix | 1 µl |
| Steril water | 39 µl |

The cycling program for the RT-PCR in a Perkin-Elmer 9600 thermocycler was as follow:

| | |
|---|---|
| 94° C 30 sec | 1 cycle |
| | |
| 94° C 5 sec | 30 cycles |
| 68° C 3 min | |

The sequences for the RT-PCR primers were generated from AAP-5 genomic sequence identified on chromosome 11.
The RT-PCR primers were as follow:

AAP-5 – 5‘primer

5‘- GGA GCC ATG GAC AAC TGT TTG GCG GCC G –3‘

AAP-5 – 3‘primer

5‘- GTG ATG GCA GTG ATG GTC AAC ATC ACA C –3‘

The PCR products were cloned into the pCR®4-TOPO vector according to the instructions of the TOPO-TA cloning® kit from Invitrogen.
The sequences of the PCR products were generated with the Applied Biosystem (ABI) Prism®BigDye™Terminator sequencing kit and analysed on a ABI 310 capillary sequencer.
The sequencing primers were the PCR primers (5' and 3') and the following:

AAP-5 - #5F

5'- ATA TTA TTC ATC TGT GCC AGA GG –3' (sense)

AAP-5 - #5R

5'-CCT CTG GCA CAG ATG AAT AAT AT –3' (antisense)

### Biochemical interactions of AAP-5

Interactions were determined by co-transfection followed by standard co-immunoprecipitation analysis exactly as described in the results section under "Co-immunoprecipitation of Myc-tagged AAP-5 protein with Apoptin in a transformed mammalian cell system".

In this case, the amount of DNA in the two-way co-transfection was 10 µg, and the partial clones were used (except AAP-3, of which a full-length sequence was used).
Pull-downs were performed by IP'ing with the appropriate AAP antibody and performing Western blot analysis with the anti-myc tag 9E10. Polyclonal antibodies directed against AAP-5, AAP-4 and AAP-3 were used.

Antibodies against AAP-3 and AAP-4 were obtained as described in the section " Generation and testing of antibodies". Peptides used for raising antibodies against AAP-3: 1) IYQRSGERPVTAGEE, 2) DEQVPDSIDAREIFD and 3) RSINDPEHPLTLEEL. Peptides used to raise antibodies against AAP-4: 1) EESTPVHDSPGKDDA, 2) DSFKTKDSFRTAKSK and 3) IDIDISSRRREDQSL.

### AAP-5 full-length: appearance and effects on tumor cells

In order to characterize the full-length AAP-5 protein, we expressed constructs encoding the full-length clones in cells and analyzed them by immunofluorescence staining. AAP-5 was analyzed as described in the results section under "Over-expression of the novel AAP-5 protein in human transformed cells induces the apoptotic process", using Saos-2 and U20S tumor cells. AAP-5 was stained with 9E10 to detect the fused myc tag. As comparison controls, Apoptin was expressed alone and in combination with AAP-5, and a construct expressing LacZ was included as a negative control. For these experiments, we examined cells after 2 and 5. days of transfection.

### Results

Apoptin induces specifically apoptosis in transformed cells, such as cell lines derived from human tumors. To identify the essential compounds in this cell-transformation-specific and/or tumor-specific apoptosis pathway, a yeast genetic screen was carried out.

We have used a human cDNA library, which is based on the plasmid vector pACT containing the complete cDNA copies made from Epstein-Barr virus-transformed human B cells (Durfee et al., 1993).

### Construction of a bait plasmid expressing a fusion gene product of GAL4-DNA-binding domain and Apoptin

To examine the existence of Apoptin-associating proteins in the human transformed/tumorigenic cDNA library, a so-called bait plasmid had to be constructed. To that end, the complete Apoptin-encoding region, flanked by about 40 base pairs downstream from the Apoptin gene, was cloned in the multiple cloning site of plasmid pGBT9.

The final construct, called pGBT-VP3, was analyzed by restriction-enzyme analysis and sequencing of the fusion area between Apoptin and the GAL4-DNA-binding domain.

### A gene(fragment) encoding an Apoptin-associating protein is determined by transactivation of a GAL4-responsive promoter in yeast.

The Apoptin gene is fused to the GAL4-DNA-binding domain of plasmid pGBT-VP3, whereas all cDNAs derived from the transformed human B cells are fused to the GAL4-activation domain of plasmid pACT. If one of the proteinaceous substances encoded by said cDNAs binds to Apoptin, the GAL4-DNA-binding domain will be in the vicinity of the GAL4-activation domain resulting in the activation of the GAL4-responsive promoter, which regulates the reporter genes HIS3 and LacZ.

The yeast clones containing plasmid expressing Apoptin and a plasmid expressing an Apoptin-associating protein fragment can grow on a histidine-minus medium and will stain blue in a beta-galactosidase assay. Subsequently, the plasmid with the cDNA insert encoding the Apoptin-associating protein can be isolated and characterized.

Before we could do so, however, we have determined that transformation of yeast cells with pGBT-VP3 plasmid alone, or in combination with an empty pACT vector, did not result in the activation of the GAL4-responsive promoter.

### Identification of Apoptin-associating protein encoded by cDNA derived from a human transformed B cell line.

We have found one yeast colony, which upon transformation with pGBT-VP3 and pACT-cDNA was able to grow on a histidine-minus medium (also lacking leucine and tryptophan) and stained blue in a beta-galactosidase assay. These results indicate that the observed yeast colony contains besides the bait plasmid pGBT-VP3 also a pACT plasmid encoding a potential Apoptin-associating protein.

Plasmid DNA was isolated from the positive yeast colony, which was transformed in bacteria. By means of a filter-hybridization assay using a pACT-specific labeled DNA-probe, the clone containing pACT plasmid could be determined. Subsequently, pACT DNA was isolated and digested with restriction enzyme *Xho*I, which resulted in the presence of an approximately 1.0-kbp cDNA insert. Finally, the cDNA insert of the pACT plasmid containing the cDNA insert was sequenced by using the Sanger method (Sanger et al., 1977).

### Description of Apoptin-associating proteins

The yeast genetic screen for Apoptin-associating proteins resulted in the detection of a cDNA clone comprising a single type of protein, namely a novel protein called Apoptin-associating protein 5, abbreviated as AAP-5.

The determined DNA sequence part of the AAP-5 cDNA clone is shown in Figure 1. The amino acid sequence, derived from the detected DNA sequence of clone AAP-5is given in Figure 2.

### Construction of an expression vector for the identification of AAP-5 protein in mammalian cells.

To study whether the cloned cDNA AAP-5 indeed encode (Apoptin-associating) a protein product, we have carried out the following experiments.

The DNA plasmid pMT2SM contains the adenovirus 5 major late promoter (MLP) and the SV40 ori enabling high levels of expression of foreign genes in transformed mammalian cells, such as SV-40-transformed Cos cells.
Furthermore, the pMT2SM vector contains a Myc-tag (amino acids: EQKLISEEDL) which is in frame with the foreign-gene product. This Myc-tag enables the recognition of e.g. Apoptin-associating proteins by means of the Myc-tag-specific 9E10 antibody.

The pMT2SM vector expressing Myc-tagged AAP-5 cDNA was constructed as follows. The pACT-AAP-5 cDNA clone was digested with the restriction enzyme *Xho*I and the cDNA insert was isolated. The expression vector pMT2SM was digested with *Xho*I and treated with calf intestine alkaline phosphatase and ligated to the isolated AAP-5 cDNA inserts. By sequence analysis, the pMT2SM constructs containing the AAP-5 cDNA in the correct orientation was identified.

The synthesis of Myc-tagged AAP-5 protein was analyzed by transfection of Cos cells with plasmid pMT2SM-AAP-5. As negative control, Cos cells were mock-transfected. Two days after transfection, the cells were lysed and Western-blot analysis was carried out using the Myc-tag-specific antibody 9E10.

The Cos cells transfected with pMT2SM-AAP-5 were proven to synthesise a specific Myc-tagged AAP-5 product with the size of approximately 18 kDa. As expected, the lysates of the mock-transfected Cos cells did not contain a protein product reacting with the Myc-tag-specific antibodies.

These results indicate that we have been able to isolate a cDNA that is able to produce a protein product with the ability to associate to the apoptosis-inducing protein Apoptin.

### Co-immunoprecipitation of Myc-tagged AAP-5 protein with Apoptin in a transformed mammalian cell system.

Next, we have analyzed the association of Apoptin and the AAP-5 protein by means of co-immunoprecipitations using the Myc-tag-specific antibody 9E10. The 9E10 antibodies were shown not to bind directly to Apoptin, which enables the use of 9E10 for carrying out co-immunoprecipitations with (myc-tagged) Apoptin-associating proteins and Apoptin.

To that end, Cos cells were co-transfected with plasmid pCMV-VP3 encoding Apoptin and with plasmid pMT2SM-AAP-5. As a negative control, cells were transfected with pCMV-VP3 expressing Apoptin and a plasmid pcDNA3.1.LacZ-myc/His-LacZ encoding the myc-tagged beta-galactosidase, which does not associate with Apoptin.

Two days after transfection, the cells were lysed in a buffer consisting of 50 mM Tris (7.5), 250 mM NaCl, 5 mM EDTA, 0.1 % Triton X100, 1 mg/ml Na₄P₂O₇ and freshly added protease inhibitors such as PMSF, Trypsine-inhibitor, Leupeptine and Na₃VO₄. The specific proteins were immuno-precipitated as described by Noteborn et al. (1998) using the Myc-tag-specific antibodies 9E10, and analyzed by Western blotting.

Staining of the Western blot with 9E10 antibodies and 111.3 antibodies, which are specifically directed against myc-tag and Apoptin, respectively, showed that the "total" cell lysates contained the 16-kDa Apoptin product and the Myc-tagged AAP-5 protein of 18 kDa. By means of a specific LacZ polyclonal antibody also the beta-galactosidase product could be visualized.

Immunoprecipitation of the Myc-tagged AAP-5 products was accompanied by the immunoprecipatation of Apoptin product of 16 kDa. In contrast, immunoprecipitation of myc-tagged beta-galactosidase did not result in a detectable co-precipitation of the Apoptin protein. In addition, immunoprecipitation of the Apoptin protein, by means of a polyclonal antibody directed against the C-terminal part of Apoptin (Noteborn and Danen, unpublished results), was accompanied by the immunoprecipitation of the AAP-5 product of 18 kDa, but not by beta-galactosidase protein.

In total, three independent immunoprecipitation experiments were carried out, which all showed the specific associating ability of Apoptin protein to the AAP-5 protein.

These results indicate that the novel determined AAP-5 protein is able to specifically associate with Apoptin not only in the yeast background, but also in a mammalian transformed cellular system.

### Over-expression of the novel AAP-5 protein in human transformed cells induces the apoptotic process.

In addition, we have examined whether AAP-5 carries apoptotic activity. First, we have analyzed the cellular localization of the novel AAP-5 protein in human transformed cells. To that end, the human osteosarcoma-derived Saos-2 cells and U20S cells were transfected, as described by Danen-van Oorschot (1997), with plasmid pMT2SM-AAP-5 encoding the myc-tagged AAP-5 protein, respectively.

By indirect immunofluorescence using the myc-tag-specific antibody 9E10 and DAPI, which stains the nuclear DNA, it was shown that AAP-5 protein was present both in the nucleus as well as in the cytoplasm of most of the tumor cells and in a minor part of the cells in the nucleus or cytoplasm alone. In cells in which both the nucleus and cytoplasm were stained with AAP-5, the nuclear staining was somewhat more positive. Co-expression of AAP-5 and Apoptin resulted in a more concrete 'thready/blobby' structural entities containing both AAP-5 and Apoptin. These structures are present mainly in the cytoplasm but also within the nucleus. These observations illustrate that AAP-5 and Apoptin clearly can co-localize with each other.

Already, three days after transfection a significant amount of Saos-2 cells and U2OS cells synthesizing AAP-5 underwent induction of apoptosis. These AAP-5-positive cells were aberrantly stained with DAPI, which is indicative for induction of apoptosis (Telford, 1992, Danen-van Oorschot, 1997). Four days after transfection, the level of apoptotic AAP-5-positive cells even increased. Cells expressing Apoptin also underwent apoptosis, whereas as expected the cells synthesizing the non-apoptotic beta-galactosidase (LacZ) protein did not. Co-expression of bothApoptin and AAP-5 proteins in human tumor cells, such as Saos-2 cells, results in a slightly faster apoptotic process as expression of Apoptin or AAP-5 protein alone. The results are shown in Figure 3.

The fact that AAP-5 protein can induce apoptosis in p53-minus Saos-2 cells indicates that AAP-5 can induce p53-independent apoptosis. These results imply that AAP-5 can be used as anti-tumor agent in cases where other (chemo)therapeutic agents will fail. Furthermore, the finding that both Apoptin and AAP-5 induce a p53-independent pathway indicates that AAP-5 fits in the Apoptin-induced apoptotic pathway.

In conclusion, we have identified an Apoptin-associating protein, namely the novel AAP-5 protein, which is mainly present in the nucleus and able to induce (p53-independent) apoptosis in human tumor cells.

### The putative BRCA-binding protein BRIP1 shows some amino-acid sequence homologies with AAP-5.

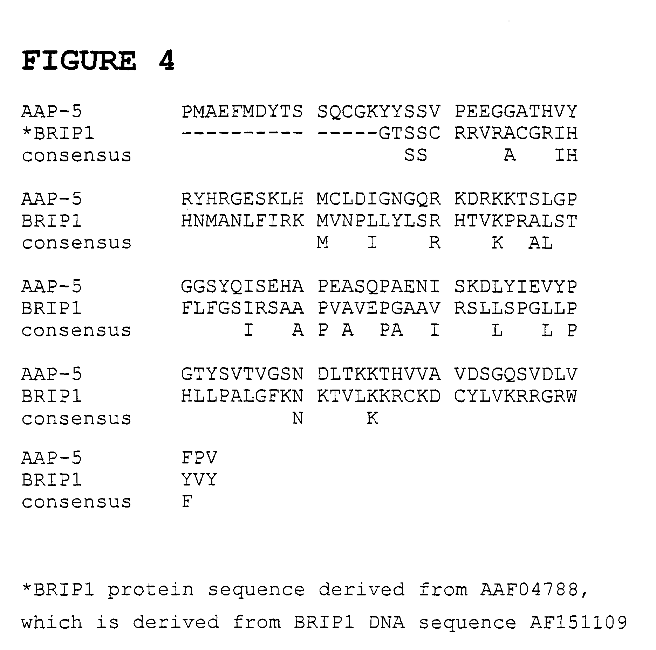

Sequence homology analysis of AAP-5 with known DNA and amino-acid sequences revealed some homology (Figure 4) with the human putative BRCA-binding protein BRIP1. The cellular BRCA protein is related with tumor formation. The fact that the Apoptin-associating protein AAP-5 shows some homology with a putative BRCA-binding protein suggests that AAP-5 also is tumor-related, which is in agreement with the finding that AAP-5 is part of the tumor-specific pathway of Apoptin-induced apoptosis.

### Utility of AAP-5 antisera

The AAP-5 antibody of the two generated with the highest affinity was selected for further use. We tested the efficacy of this antibody by transfecting primate COS-7 and human U2OS cells with the partial AAP-5-myc construct.

With AAP-5-myc DNA transfections, Western analysis showed that an approximately 17 or 12 kD (COS and U2OS, respectively) AAP-5-myc protein was detected strongly only in samples where the DNA was transfected. (These size differences may be a result of cell-type-specific post-translational modifications.) Similarly, in immunoprecipitation experiments, AAP-5-myc was also strongly detected. Finally, we could detect the presence of transfected AAP-5-myc diffusely in the cytoplasm and nucleus of human Saos-2 tumor cells using the AAP-5 antibody in immunofluorescence analysis.

### Northern blot analysis

Whereas AAP-5 is not detectably expressed in the fetal tissue RNA examined, there is an approximately 1.8-2 kb message expressed in adult lung. This mRNA size is consistent with what is predicted to be expressed from the full-length gene, including post-transcriptional modifications.

### Biochemical interactions of AAP-5 withother Apoptin-associating proteins

The genetic yeast screen with pGBT-VP3 as bait plasmid and pACT plasmid containing cDNAs from transformed human B cells also delivered other Apoptin-associating proteins, which also encode Apoptin-associating proteins. These Apoptin-associating proteins were called AAP-3 and AAP-4 (see co-pending application EP01200163.2 and EP00204396.6 respectively, which are incorporated herein by reference). The DNA sequence of AAP-3 is shown in Figure 5, whereas the AAP-3 cDNA-encoded amino acid sequence is shown in figure 6. The DNA sequence of AAP-4 is shown in Figure 7, whereas the AAP-4 cDNA-encoded amino acid sequence is shown in figure 8. Just like AAP-5, both AAP-3 and AAP-4 are able to associate with Apoptin not only in the yeast background, but also in a mammalian transformed cellular system.

Immunofluorescense assays of human transformed Saos-2 cells and normal diploid VH10 fibroblasts expressing AAP-3 revealed that AAP-3 is located in both cell types predominantly in the cytoplasm and nucleus, but in lower percentages also mainly in the nucleus or mainly in the cytoplasm. Co-synthesis of AAP-3 and Apoptin in both cell types showed a clear nuclear localization of AAP-3 and Apoptin. Tumor cells that have become apoptotic showed a nuclear localization of Apoptin and a peri-nuclear staining pattern of AAP-3. As expected, normal diploid VH10 cells synthesizing both Apoptin and AAP-3 did not undergo apoptosis.

AAP-4 is present in the nucleus and able to induce (p53-independent) apoptosis in human tumor cells.

To study whether other Apoptin-associating proteins can also bind to AAP-5 multiple experiments were performed. First, we tested the ability of AAP-5 to bind to AAP-3 in co-IP analysis, and found that these two proteins do interact under these conditions. However, we showed that AAP-5 does not bind to AAP-4 (even though AAP-3 does bind to AAP-4) under these conditions. These results show that many Apoptin-associating proteins can exist in heterocomplexes.

### Cloning and sequence analysis of full-length AAP-5

A further sequence analysis of the human AAP-5 sequence yielded the 974 bp long nucleic acid sequence as shown in Figure 9. An open reading frame was found in this nucleic acid sequence at position 91 to 795. The deduced amino acid sequence is given in Figure 10.

### AAP-5: appearance and effects on tumor cells

Full-length AAP-5 in both Saos-2 and U2OS cells exhibited a primarily cytoplasmic localization. The staining was diffuse, but in addition most of the cells also contained very prominent bodies in the cytoplasm, clustering in particular around the nucleus. These bodies had a semi-regular, almost crystalline structure, and tended to cluster together into larger aggregates. In some cells a faint nuclear, diffuse staining could also be discerned. When co-expressed with Apoptin, it appeared that Apoptin formed a "shell" around the outside of the crystalline-like bodies, and the overall number of these bodies appeared to be decreased compared to the situation with the AAP-5 expressed alone.

We also examined the apoptosis potential of the full-length AAP-5 in both Saos-2 and U2OS cells. Compared to the lacZ negative control, AAP-5 caused a very high percentage of apoptosis in both cell types, almost twice the level as that seen for Apoptin alone at the same time point (2 days after transfection). This apoptosis only increased at later time points, similar to what was seen with the partial AAP-5 protein. Also similar to the partial AAP-5 protein, when co-expressed with Apoptin, there was a slight increase in the amount of death as compared to either protein expressed alone.

The apoptosis inducing capability of the partial AAP-5 protein or the full-length AAP-5 protein alone or in combination with Apoptin shows that all possible mutants ranging from at least the partial AAP-5 protein to the full-length AAP-5 protein are capable of inducing apoptosis. Therefore, deletion mutants of the full-length AAP-5 protein comprising at least the partial AAP-5 protein are capable of inducing apoptosis. Also, point mutants (both at nucleotide and amino acid level) of the full-length AAP-5 protein comprising at least the partial AAP-5 protein are capable of inducing apoptosis.

### Expression and detection of full-length AAP-5 on Western blot.

Western analysis of the full-length AAP-5 protein expressed in COS-cells followed by ECL analysis using the anti-myc tag antibody 9E10. showed a specific band consistent with the predicted size (30 kDa) of the full-length AAP-5.

### Description of the figures

Figure 1 shows the partial sequence of vector pMT2SM-AAP-5.

Figure 2 shows the amino-acid sequence of the analyzed region of the Apoptin-associating clone AAP-5. In addition, the three C-terminal amino acids H-E-G (bold) of the multiple cloning site of pACT are given to illustrate that the AAP-5 amino acid sequence is in frame with the GAL4-activation domain. This feature proves that the AAP-5 region is indeed synthesized in yeast cells.

Figure 3 shows the apoptotic activity of AAP-5 protein,, with or without co-synthesis of Apoptin in human osteosarcoma-derived Saos-2 cells. As positive control, Saos-2 cells were transfected with a plasmid encoding Apoptin. As negative control, cells were transfected with a plasmid encoding lacZ.
(-): no apoptotic activity; (+): apoptotic activity; (++): strong apoptotic activity; (+++): very strong apoptotic activity.
In total three independent experiments have been carried out.

Figure 4 shows the partial consensus amino-acid sequences of AAP-5 and the putative BRCA-binding protein BRIP-1.

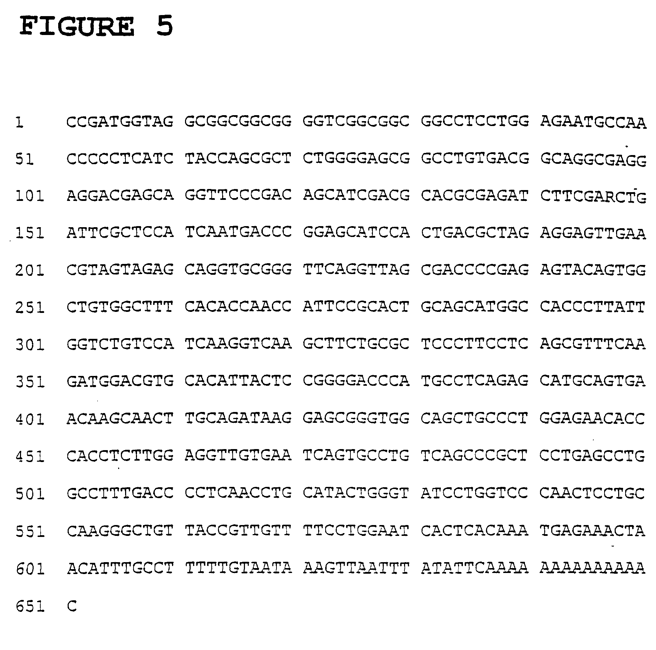

Figure 5 shows the partial sequence of vector pMT2SM-AAP-3

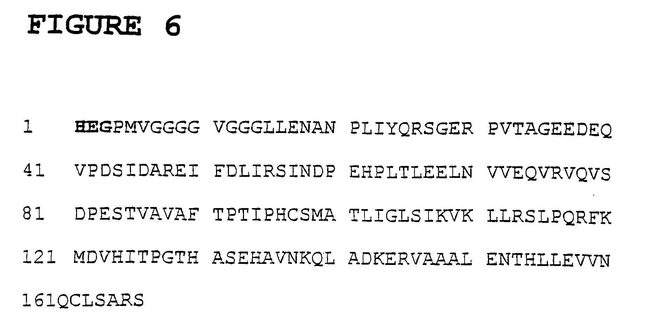

Figure 6 shows the amino acid sequence of the analysed region of the Apoptin-associating clone AAP-3. In addition to, the three C-terminal amino acids H-E-G (bold) of the multiple cloning site of pact are given to illustrate that the AAP-3 amino acid sequence is in frame with the GAL4-activation domain. This feature proves that the AAP-3 region is indeed synthesized in yeast cells.

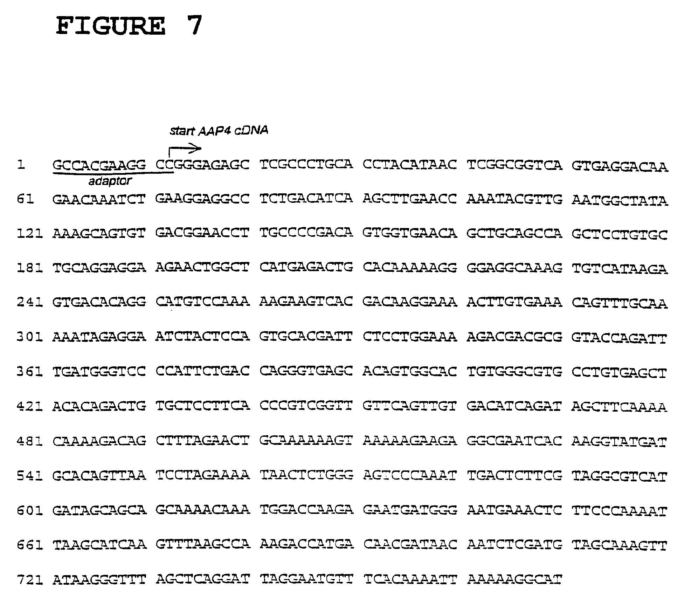

Figure 7 shows the partial sequence of vector pMT2SM-AAP-4. The DNA sequence of the AAP-4 CDNA starts at position 12 of the DNA sequence and is indicated as "start AAP-4 cDNA".

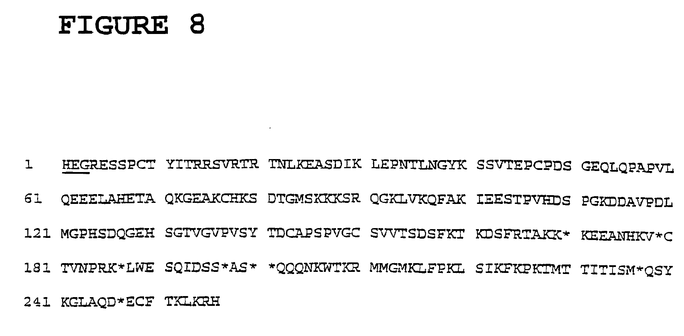

Figure 8 shows the amino acid sequence of the analysed region of the Apoptin-associating clone AAP-4. In addition to, the three C-terminal amino acids H-E-G (bold) of the multiple cloning site of pact are given to illustrate that the AAP-4 amino acid sequence is in frame with the GAL4-activation domain. This feature proves that the AAP-4 region is indeed synthesized in yeast cells.

Figure 9 shows the nucleic acid sequence of full-length AAP-5. The ATG start codon is at position 91-93 and the TAA stop codon is at position 793-795.

Figure 10 shows the amino acid sequence deduced from the nucleic acid sequence of Figure 9.

### REFERENCES

1. Bellamy, C.O.C., Malcomson, R.D.G., Harrison, D.J., and Wyllie, H. 1995. Cell death and disease: The biology and regulation of apoptosis. Seminars in Cancer Biology 6, 3-12.
2. Danen-Van Oorschot, A.A.A.M., Fischer, D.F., Grimbergen, J.M., Klein, B., Zhuang, S.-M., Falkenburg, J.H.F., Backendorf, C., Quax, P.H.A., Van der Eb, J.A., and Noteborn, M.H.M. (1997). Apoptin induces apoptosis in human transformed and malignant cells but not in normal cells. Proceedings National Academy Sciences, USA: 94, 5843-5847.
3. Danen-Van Oorschot, A.A.A.M, Den Hollander, A., Takayama, S., Reed, J., Van der Eb, A.J. and Noteborn, M.H.M. (1997a). BAG-1 inhibits p53-induced but not Apoptin-induced apoptosis. Apoptosis 2, 395-402.
4. Duke, R.C., Ocjius, D.M., Young, J, D-E. (1996). Cell suicide in health and disease. Scientific American December 1996, 48-55.
5. Durfee, T., Becherer, K., Chen, P.-L., Yeh,S.-H., Yang, Y., Kilburn, A.E., Lee, W.-H., and Elledge, S.J. (1993). The retinoblastoma protein associates with the protein phosphate type I catalytic subunit. Genes and Development 7, 555-569.
6. Earnshaw, W.C., 1995. Nuclear changes in apoptosis. Current Opinion in Cell Biology 7, 337-343.
7. Fields, S. and Song, O.K. (1989). A novel genetic system to detect protein-protein interactions. Nature 340, 245-246.
8. Hockenberry, D.M. (1994). Bcl-2 in cancer, development and apoptosis. Journal of Cell Science, Supplement 18, 51-55.
9. Hoffman, C.S. and Winston, F. (1987). A ten-minute DNA preparation from yeast efficiently releases autonomous plasmids for transformation of Escherichia coili. Gene 57, 267-272.
10. Kerr, J.F.R., Winterford, C.M., and Harmon, B.V. (1994). Apoptosis: Its significance in cancer and cancer therapy. Cancer 73, 2013-2026.
11. Klebe, R.J., Harriss, J.V., Sharp, Z.D., and Douglas, M.G. (1983). A general method for polyethylene-glycol-induced genetic transformation of bacteria and yeast. Gene 25, 333-341.
12. Levine, A.J. (1997). p53, the cellular gatekeeper for growth and division. Cell 88, 323-331.
13. Maniatis, T., Fritsch, E.F., and Sambrook, J. (1982). Molecular Cloning: A Laboratory Manual. CSHL Press, New York, USA.
14. McDonell T.J., Meyn, R.E., Robertson, L.E. (1995). Implications of apoptotic cell death regulation in cancer therapy. Seminars in Cancer Biology 6, 53-60.
15. Noteborn, M.H.M. (1996). PCT application WO 96/41191. Apoptin induces apoptosis in human transformed and malignant cells but not in normal cells as essential characteristic for the development of an anti-tumor therapy.
16. Noteborn, M.H.M., and De Boer, G.F. (1996). Patent USA/no. 030, 335.
17. Noteborn, M.H.M., De Boer, G.F., Van Roozelaar, D., Karreman, C., Kranenburg, O., Vos, J., Jeurissen, S., Zantema, A., Hoeben, R., Koch, G., Van Ormondt, H., and Van der Eb, A.J. (1991). Characterization of cloned chicken anemia virus DNA that contains all elements for the infectious replication cycle. Journal of Virology 65, 3131-3139.
18. Noteborn, M.H.M., and Pietersen, A. (1998). A gene delivery vehicle expressing the apoptosis-inducing proteins VP2 and/or Apoptin. PCT Application no. PCT/NL98/00213
19. Noteborn, M.H.M., Todd, D., Verschueren, C.A.J., De Gauw, H.W.F.M., Curran, W.L., Veldkamp, S., Douglas, A.J., McNulty, M.S., Van der Eb, A.J., and Koch, G. (1994). A single chicken anemia virus protein induces apoptosis. Journal of Virology 68, 346-351.
20. Noteborn, M.H.M., Verschueren, C.A.J., Koch, G., and Van der Eb, A.J. (1998). Simultaneous expression of recombinant baculovirus-encoded chicken anemia virus (CAV) proteins VP1 and VP2 is required for formation of the CAV-specific neutralizing epitope. Journal General Virology, 79, 3073-3077.
21. Noteborn, M.H.M., and Zhang, Y. (1998). Methods and means for determining the transforming capability of agents, for determining the predisposition of cells to become transformed and prophylactic treatment of cancer using Apoptin-like activity. PCT Application no. PCT/NL98/00457
22. Noteborn, M.H.M., Danen-van Oorschot, A.A.A.M., Van der Eb, A.J. (1998a). Chicken anemia virus: Induction of apoptosis by a single protein of a single-stranded DNA virus. Seminars in Virology 8, 497-504.
23. Paulovich, A.G., Toczyski, D., Hartwell, H. (1997). When checkpoints fail. Cell 88, 315-321.
24 Pietersen, A.M., Van der Eb, M.M., Rademaker, H.J., Van den Wollenberg, D.J.M., Rabelink, M.J.W.E., Kuppen, P.J.K., Van Dierendonck, J.H., Van Ormondt, H., Masman, D., Van de Velde, C.J.H., Van der Eb, Hoeben, R.C., and Noteborn, M.H.M. (1998). Specific tumor-cell killing with adenovirus vectors containing the Apoptin gene. Gene Therapy 6, 882-892.
25. Rose, M.D., Winston, F., and Hieter, P. (1990). Methods in yeast genetics. A laboratory course manual. Cold Spring Harbor Laboratory Press, Cold Spring Harbor, New York, USA.
26. Sachs, L. and Lotem, J. (1993). Control of programmed cell death in normal and leukemia cells: New implications for therapy. Blood 82, 15-21.
27. Sanger, F., Nicklen, S., and Coulsen, A.R. (1977). DNA sequencing with chain-terminating inhibitors. Proceedings National Academic Sciences USA 74, 5463-5467.
28. Steller, H. (1995). Mechanisms and genes of cellular suicide. Science 267, 1445-1449.
29. Telford, W.G., King, L.E., Fraker, P.J. (1992). Comparative evaluation of several DNA binding dyes in the detection of apoptosis-associated chromatin degradation by flow cytometry. Cytometry 13, 137-143.
30. Teodoro, J.G. and Branton, P.E. (1997). Regulation of apoptosis by viral gene products. Journal of Virology 71, 1739-1746.
31. Thompson, C.B. (1995). Apoptosis in the pathogenesis and treatment of disease. Science 267, 1456-1462.
32. White, E. (1996). Life, death, and the pursuit of apoptosis. Genes and development 10, 1-15.
33. Wyllie, A.H. (1995). The genetic regulation of apoptosis. Current Opinion in Genetics and Development 5, 97-104.
34. Wyllie, A.H., Kerr, J.F.R., Currie, A.R. (1980). Cell death: The significance of apoptosis. International Review of Cytology 68, 251-306.
35. Yang, X., Hubbard, E.J.A., and Carlson, M. (1992). A protein kinase substrate identified by the two-hybrid system. Science 257, 680-682.
36. Zhuang, S.-M., Landegent, J.E., Verschueren, C.A.J., Falkenburg, J.H.F., Van Ormondt, H., Van der Eb, A.J., Noteborn, M.H.M. (1995). Apoptin, a protein encoded by chicken anemia virus, induces cell death in various human hematologic malignant cells *in vitro.* Leukemia 9 S1, 118-120.
37. Zhuang, S.-M., Shvarts, A., Van Ormondt, H., Jochemsen, A.-G., Van der Eb, A.J., Noteborn, M.H.M. (1995). Apoptin, a protein derived from chicken anemia virus, induces a p53-independent apoptosis in human osteosarcoma cells. Cancer Research 55, 486-489.
38. Noteborn et. al. (2000) European patent application 01200163.2, Apoptin-associating protein
39. Noteborn et al. (2000) European patent application 00204396.6, Apoptin-associaitng protein
40. Evans GI, Lewis GK, Ramsay G, Bishop JM (1985), Isolation of monoclonal antibodies specific for human c-myc proto-oncogene product. Mol Cell. Biol. Dec 5 (12), 3610-3616

## Claims

1. An isolated or recombinant nucleic acid as shown in fig. 1 or fig.9 encoding an Apoptin-associating proteinaceous substance capable of providing apoptosis or a functional equivalent or functional fragment thereof.

2. A nucleic acid according to claim 1 derived from a cDNA library.

3. A nucleic acid according to claim 1 or 2 wherein said cDNA library comprises human cDNA.

4. A nucleic acid according to anyone of claims 1 to 3 capable of hybridising to a nucleic acid molecule encoding an Apoptin-associating proteinaceous substance as shown in figures 1 and 9.

5. A nucleic acid according to anyone of claims 1 to 4 being at least 60% homologous to a nucleic acid molecule, or to a functional equivalent or functional fragment thereof, encoding an Apoptin-associating proteinaceous substance as shown in figure 1 and/or 9.

6. A vector comprising a nucleic acid according to anyone of claims 1 to 5.

7. A vector according to claim 6 comprising a gene-delivery vehicle.

8. A host cell comprising a nucleic acid according to anyone of claims 1 to 5 or a vector according to claim 6 or 7.

9. A host cell according to claim 8 which is a eukaryotic cell such as a yeast cell or a vertebrate cell.

10. An isolated or recombinant Apoptin-associating proteinaceous substance comprising a sequence as shown in fig.2 or fig. 10 or a functional equivalent thereof capable of providing apoptosis.

11. A proteinaceous substance according to claim 10 encoded by a nucleic acid according to anyone of claims 1 to 5.

12. A proteinaceous substance according to claim 10 or 11 comprising at least a part of an amino acid sequence as shown in figure 2 or 10 or a functional equivalent or functional fragment thereof.

13. An isolated or synthetic antibody specifically recognising a proteinaceous substance or functional equivalent or functional fragment thereof according to anyone of claims 10 to 12.

14. A proteinaceous substance specifically recognisable by an antibody according to claim 13.

15. Use of a nucleic acid according to anyone of claims 1 to 5, a vector according to claims 6 or 7, a host cell according to claim 8 or 9, a proteinaceous substance according to anyone of claims 10 to 12 or 14 for the induction of apoptosis.

16. Use according to claim 15 wherein said apoptosis is p53-independent.

17. Use according to claim 15 or 16 further comprising use of a nucleic acid encoding Apoptin or a functional equivalent or fragment thereof or use of Apoptin or a functional equivalent or fragment thereof.

18. A pharmaceutical composition comprising a nucleic acid according to anyone of claims 1 to 5, a vector according to claims 6 or 7, a host cell according to claim 8 or 9, a proteinaceous substance according to anyone of claims 10 to 12 or 14.

19. A pharmaceutical composition according to claim 18 further comprising a nucleic acid encoding Apoptin or a functional equivalent or fragment thereof or Apoptin or a functional equivalent or fragment thereof.

20. A pharmaceutical composition according to claim 18 or 19 for the induction of apoptosis.

21. A pharmaceutical composition according to claim 20 wherein said apoptosis is p53-independent.

22. A pharmaceutical composition according to anyone of claims 18 to 21 for the treatment of a disease where enhanced cell proliferation or decreased cell death is observed.

23. A pharmaceutical composition according to claim 22 wherein said disease comprises cancer or auto-immune disease.

24. A method for treating an individual carrying a disease where enhanced cell proliferation or decreased cell death is observed comprising treating said individual with a pharmaceutical composition according to anyone of claims 18 to 23.
